# EUROPÄISCHE PATENTSCHRIFT

(11) **EP 0 288 653 B1**
(45) Veröffentlichungstag und Bekanntmachung des Hinweises auf die Patenterteilung: **10.03.1993**
(21) Anmeldenummer: 87890252.7
(22) Anmeldetag: 11.11.1987
(51) Int. Cl.: G01N 33/38, G01N 11/10, G01N 3/42

(54) **Verfahren zum Prüfen des Erstarrungsverhaltens bzw. der Erstarrungsgeschwindigkeit von zementgebundenen Massen und Vorrichtung zur Durchführung des Verfahrens**
Method for testing the setting behaviour or rate of cement-bound masses, and apparatus for carrying out the method
Procédé pour tester le comportement et la vitesse de prise de masses liées par du ciment et dispositif pour la mise en oeuvre du procédé

(30) Priorität: 29.04.1987 AT 1065/87
(43) Veröffentlichungstag der Anmeldung: 02.11.1988
(73) Patentinhaber: Mayreder, Keil, List u. Co. Baugesellschaft m.b.H., A-8010 Graz (AT)
(72) Erfinder: Roth, Hubert, Prof.Ing., A-8020 Graz (AT); Fleischmann, Alfred, Dipl.Ing., A-8043 Graz (AT)
(74) Vertreter: Hübscher, Heiner, Dipl.-Ing.

(56) Entgegenhaltungen:
- EP-A- 0 125 774
- FR-A- 1 557 095
- DEUTSCHES INSTITUT FÜR NORMEN, NORMENAUSSCHUSS BAUWESEN, November 1978, Beuth Verlag GmbH, Berlin, DE, DIN 1164, Teil 5, "Portland-, Eisenportland-, Hochofen- und Trasszement: Bestimmung der Erstarrungszeit mit dem Nadelgerät"

## Beschreibung

Die Erfindung betrifft ein Verfahren zum Prüfen des Erstarrungsverhaltens bzw. der Erstarrungsgeschwindigkeit von zementgebundenen Massen, insbesondere bei der Herstellung von Spritzbeton, bei dem die Eindringtiefe einer mit vorgewählter Kraft an eine aus der zu prüfenden Masse gebildeten Probe angestellten Penetrationsnadel als Maß für den momentanen Erstarrungszustand verwendet wird und unter vergleichbaren Bedingungen hergestellte bzw. gewonnene Proben in becherartigen Prüfgefäßen in den Verstellbereich der Nadel verbracht, die von oben in das Prüfgefäß tauchende Nadel in festgelegten Zeitabständen in abstandsweise vorgesehene Einstichstellen eindringen gelassen und die der Eindringtiefe entsprechenden Daten erfaßt werden.

Ein derartiges Verfahren ist in seinen Grundzügen in DEUTSCHES INSTITUT FÜR NORMEN, NORMENAUSSCHUSS BAUWESEN, November 1978 Beuth Verlag GmbH, Berlin, DE, DIN 1164 Teil 5 "Portland-, Eisenportland-, Hochofen- und Trasszement: Bestimmung der Erstarrungszeit mit dem Nadelgerät" vorbeschrieben. Dabei wird bisher ein einfaches von Hand aus zu bedienendes Nadelgerät verwendet und die mit seiner Hilfe ermittelten Daten werden aufgezeichnet, wobei auch die Zeitabstände zwischen Erstellung der Probe und den Eindringungszeiten für die Nadel aus der Ablesung entsprechender Uhren zu ermitteln sind und eingehalten werden sollen. Der Widerstand, den die zementgebundene Masse zu einem bestimmten Zeitpunkt nach Beginn des Erstarrungsvorganges dem Eindringen der Penetrationsnadel entgegensetzt und somit die Eindringtiefe dieser Nadel während einer bestimmten Zeit ergeben an sich ein gutes Maß für den momentanen Erstarrungszustand. Man kann auch die Nadel mit einer vorbestimmten Kraft anstellen und die Eindringtiefe messen. Für die fortlaufende Überwachung der Güte bzw. Eigenschaften von Füll- und Spritzbeton ist das Verfahren in seiner bisherigen Verwirklichung allerdings weniger geeignet, da die bekannten Vorrichtungen mit Penetrationsnadel im wesentlichen für den Laboreinsatz bestimmt sind. Bei Spritz-oder Füllbeton beeinflußt das Erstarrungsverhalten bei der Verarbeitung unter anderem die Güte der Verbindung von nacheinander aufgetragenen Betonschichten, von aneinandergrenzenden Betonschichten und die Schichtdicke mit der in einem Auftragungsvorgang gearbeitet werden kann, ohne daß die Gefahr einer Ablösung einer zu dicken Schicht vom Haftgrund besteht. Bei Füllbeton beeinflußt das Erstarrungsverhalten die durch den Druck des eingefüllten Betons auf eine Schalung ausgeübten Kräfte und bei der Verwendung von Gleitschalungen damit auch die Dimensionierung und zulässige Verstellgeschwindigkeit der Gleitschalung. Im Labor gewonnene Erkenntnisse über das Erstarrungsverhalten stimmen häufig mit dem tatsächlichen Verhalten von in richtiger Mischung eingesetzten und verarbeiteten Betonmassen nur bedingt überein, wobei auch die momentan wirksamen Faktoren z. B. Zementqualität, Mischungsverhältnis, Wasserzementwert, Art, Menge und Verträglichkeit verwendeter Verzögerungsmittel und die momentan eingesetzten Zuschlagstoffe nicht voll berücksichtigt werden können.

Es wurde schon vorgeschlagen, eine Penetrationsnadel an einem einer Nähmaschine ähnlichem Gerät anzubringen, mit ihrer Hilfe in eine Probe fortlaufend Einstiche zu machen und aus der sich verringernden Einstichtiefe Rückschlüsse auf das Erstarrungsverhalten der Probe zu ziehen, doch ist auch dieses Verfahren praktisch auf die Laboranwendung beschränkt.

Aus der EP-A-0 125 774 sind ein Verfahren und ein zu seiner Durchführung geeignetes Gerät für die Betonuntersuchung bekannt, mit dessen Hilfe Betonproben weitgehend automatisiert untersucht werden können. Dabei wird aus frisch gemischtem Beton durch Absiebung der Kiesanteil entfernt und der aus den Feinbestandteilen und dem Zementleim bestehende Mörtel wird mit Wasser in vorbestimmter Menge versetzt, aufgerührt, eine bestimmte Zeit stehengelassen und dann weiter untersucht, wobei unter anderem die Dichte des Mörtels durch Eintauchen des sich in einem Gefäß aus der Mörtel-Wassermischung absetzenden Festbestandteiles durch Wägung ermittelt wird. Die Wägung der Festbestandteile wird vor dem Aufschlämmen mit Wasser in der Luft ebenfalls vorgenommen. Aus den Meßdaten lassen sich Rückschlüsse auf das Zement-Wasserverhältnis im fertigen Beton ziehen. Die verwendete Vorrichtung ermöglicht eine Automatisierung der aufgezeigten Vorgänge, wobei ein angeschlossener Rechner den Verfahrensablauf steuert und auch gleich Daten ausgibt, die z. B. angeben, welche Festigkeit für den Beton zu erwarten ist bzw. wie die Zuschlagstoffe oder das Mischungsverhältnis des Betons zu ändern sind, um die erwünschte Qualität zu erhalten.

Aufgabe der Erfindung ist es, ein Verfahren der eingangs genannten Art zu verbessern, um seiner Hilfe exakte und wiederholbare Untersuchungen des Erstarrungsverhaltens unter vergleichbaren Bedingungen schnell und weitgehend automatisiert auch unmittelbar auf Baustellen durchführen zu können.

Die gestellte Aufgabe wir durch die Merkmale des Patentanspruches gelöst.

Das beanspruchte Verfahren ist somit gekennzeichnet durch die Verwendung einer Vorrichtung, die unterhalb einer Vertikalführung (9) für einen die Penetrationsnadel (12) haltenden, durch sein Eigengewicht und wahlweise anbringbare Zusatzgewichte (11) belasteten Nadelträger (10) eine z. B. tellerförmige Stütze (7) für je ein becherartiges Prüfgefäß (8) aufweist, welche Stütze (7) horizontal gegenüber dem Nadelträger (10) einstellbar und um ihre Achse verdrehbar ist, wobei für den Nadelträger (10) eine Hubeinrichtung (13) und eine Auslöseeinrichtung (14) zur Freigabe seiner freien Abwärtsbewegung sowie eine Wege-Meßeinrichtung (15, 16), für die Stütze (7) Antriebseinrichtungen (5, 6) zu ihrer Drehverstellung und Horizontaleinstellung vorgesehen sind und eine mit einem Rechner, insbesondere einem frei programmierbaren Personalcomputer ausgestattete Steuer- und Auswerteeinheit (17) die Hubeinrichtung (13), Auslöseeinrichtung (14) und Antriebseinrichtungen (5, 6) zyklisch steuert, so daß die Nadel (12) bei einem Zyklus im freien Fall in eine in dem auf der Stütze (7) befindlichen Prüfgefäß (8) enthaltene Probe (22) eindringt, die Eindringtiefe über die Wege-Meßeinrichtung (15, 16) von der Auswerteeinheit (17) erfaßt, die Nadel (12) angehoben, das Prüfgefäß (8) über die Antriebseinrichtungen (5, 6) verstellt und dann die Nadel (12) im Zeittakt neuerlich ausgelöst wird, so daß sie beim nächsten Zyklus in eine im Abstand von der letzten Einstichstelle befindliche Einstichstelle der Probe (22) eindringt, die Probe (22) nach Abschluß der Untersuchung in einem vorbestimmten Muster, z. B. mit gleichen Abständen auf konzentrischen Kreisen (23, 24, 25), Einstichstellen aufweist und in der Auswerteeinheit (17) ein auswertbares Datenprofil für die erfaßten Eindringtiefen gespeichert ist.

Das mit der beim erfindungsgemäßen Verfahren verwendeten Vorrichtung von jeder Probe erhaltene Datenprofil wird jeweils unter mit anderen Untersuchungen voll vergleichbaren Bedingungen erzeugt, so daß es auch die Erstarrungsgeschwindigkeit umfaßt und mit einem Solldatenprofil, das einem für den jeweiligen Anwendungsfall idealen Datenprofil entspricht, vergleichbar ist. Dabei können zulässige Toleranzen vorgegeben werden, deren Überschreitung anzeigt, daß eine Änderung in der Zusammensetzung der zementgebundenen Massen erforderlich erscheint bzw. daß das geänderte Erstarrungsverhalten während des momentanen Anwendungsfalles beachtet werden muß. Die Ausgabe der Daten kann in Kurvendarstellungen auf einem Bildschirm, durch Ziffernangaben oder durch Ausdrucke über einen vom Rechner gesteuerten Drucker erfolgen. Es können anwendungsspezifische Auswertungsprogramme gewählt werden. Das Verfahren kann sowhl bei etwa an einer Baustelle aus dem eben verarbeiteten Beton gezogenen Proben als auch an gesondert hergestellten Proben angewendet werden.Für die Proben werden bevorzugt glatte Kunststoffgefäße verwendet, die eine nachträgliche Entformung erleichtern. Die entformten Proben können als Rohlinge für Festigkeitsüberprüfungen eingesetzt werden. Zur Berücksichtigung unterschiedlicher Füllhöhen bei den Prüfgefäßen ist es möglich, die Nadel vor Beginn des Prüfvorganges auf die Oberfläche des Prüflings einzustellen und diesen Einstellwert als Nullwert der Eindringtiefe im Rechner zu speichern. Man kann aber auch zusätzlich eine Höhenverstellung der Stütze vorsehen und über diese Höhenverstellung die Probenoberfläche in eine vorgewählte Einstellage bringen.

Weitere Einzelheiten und Vorteile des Erfindungsgegenstandes entnimmt man der nachfolgenden Zeichnungsbeschreibung.

In der Zeichnung ist der Erfindungsgegenstand beispielsweise veranschaulicht. Es zeigt
- Fig. 1: eine Vorrichtung zum weitgehend automatisierten Prüfen des Erstarrungsverhaltens von zementgebundenen Massen in stark schematisierter Darstellungsweise in Seitenansicht und
- Fig. 2: eine Probe nach dem Prüfvorgang in Draufsicht.

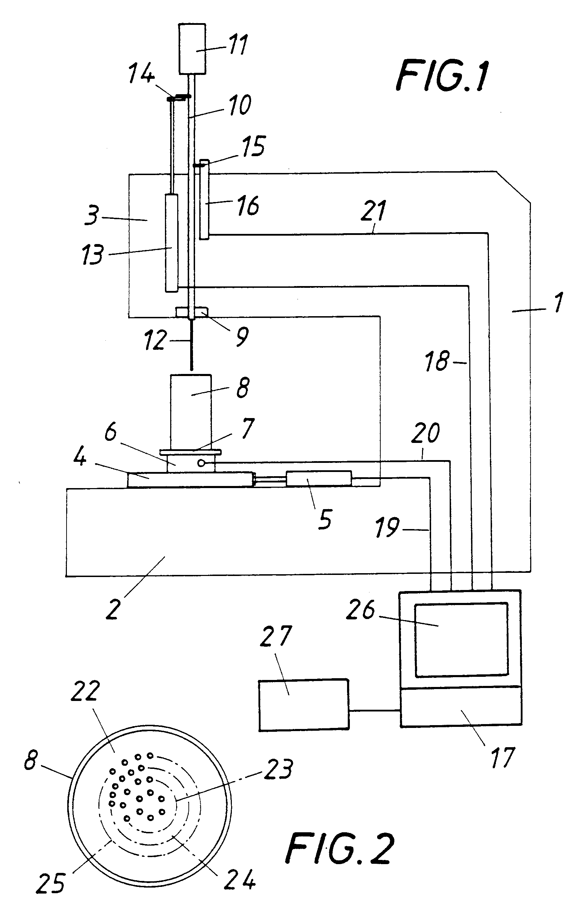

Das Gerät nach Fig. 1 besitzt ein nur in seinen Umrissen veranschaulichtes Gehäuse 1 mit einem Fußteil 2 und einem im Abstand oberhalb des Fußteiles angeordneten Ausleger 3. Am Fußteil 2 ist ein Schlitten 4 mit Hilfe eines pneumatischen Kolbentriebes 5 in Längsrichtung des Fußteiles verstellbar angebracht. Der Schlitten trägt einen Motor 6, der wahlweise als elektrischer Schrittmotor oder auch als pneumatischer Motor ausgebildet sein kann und der eine als Teller 7 dargestellte Stütze antreibt, auf der ein becherartiges Prüfgefäß 8 abgestützt ist, das bis zu einer vorgewählten Füllhöhe mit einer Probe aus der zu prüfenden zementgebundenen Masse gefüllt ist. Im Ausleger 3 befindet sich eine Vertikalführung 9 für einen Nadelträger 10, der durch sein Eigengewicht und wahlweise aufsteckbare Zusatzgewichte 11 belastet ist und der eine Penetrationsnadel 12 trägt. Eine ebenfalls aus einem pneumatischen Kolbentrieb bestehende Hubeinrichtung 13 ist über eine auslösbare Schaltklinke 14 mit dem Nadelträger 10 verbunden. Bei Auslösen der Schaltklinke 14 kann der Nadelträger mit der Nadel 12 in einem dem freien Fall vergleichbaren Zustand nach unten wandern, wobei die Nadel 12 in die im Gefäß 8 enthaltene Probe eindringt.

Mit dem Nadelträger 10 ist noch ein Fühler 15 einer Längenmeßeinrichtung 16 verbunden, die als Linearpotentiometer ausgeführt sein kann. Es ist aber auch möglich, eine berührungslos arbeitende Längenmeßeinrichtung mit einem nach optoelektronischen, kapazitiven oder induktiven Abtastprinzipien von einer mit dem Fühler 15 bewegten Abtasteinheit abgetasteten Maßstab, vorzugsweise einem Inkrementalmaßstab zu verwenden. Die Druckluftzuleitungen wurden der besseren Übersichtlichkeit halber weggelassen. Dem Gerät 1 zugeordnet ist eine kombinierte Steuer- und Auswerteeinheit in Form eines frei programmierbaren Personalcomputers 17 vorgesehen, die gegebenenfalls über Schnittstellen und Steuerleitungen 18, 19, 20 die Antriebseinrichtungen 5, 7, die Hubeinrichtung 13 und gegebenenfalls den Auslösemechanismus 14 steuert. Ferner erhält die Einheit 17 von der Längenmeßeinrichtung über eine Leitung 21 Meßsignale entsprechend dem Verstellweg des Fühlers 15.

Bei einer Untersuchung wird in das Prüfgefäß 8 eine Probe des zu untersuchenden Materials eingebracht, die beispielsweise einer laufenden Charge von eben verarbeitetem Spritzbeton bzw. dem dabei eingesetzten Zementleim mit den zugesetzten Beschleunigermitteln entnommen wird. Eine andere Möglichkeit besteht darin, unmittelbar im Prüfgefäß 8 eine entsprechende Masse aus proportional eingesetzten Bestandteilen zu mischen, wobei vorzugsweise zunächst Zement und allfällige Zuschläge trocken vorgemischt und dann erst Wasser und Beschleuniger zugegeben und für eine definierte Zeitspanne, beispielsweise 10 Sekunden lang, mit einem Rührgerät, z. B. einem Korbmischer, eingemischt werden, wonach das Prüfgefäß 8 unmittelbar auf die Stütze 7 aufgestellt wird. Nach einer Variante kann eine entsprechende Rühreinrichtung auch am Gerät 1 angebracht sein, wobei der Ausleger 1 oder der Fußteil 2 verschwenkbar sind, so daß wahlweise die Rühreinrichtung oder die Nadel 12 über das Gefäß 8 eingestellt werden. Man kann die Rühreinrichtung auch neben der Nadelführung am Arm 3 höhenverstellbar vorsehen und den Schlitten 4 über den dann entsprechend lang ausgebildeten Kolbentrieb 5 nach dem Mischvorgang aus der Rührstation in die dargestellte Prüfstation verstellen. Hier kann die Zeit zwischen dem Ende des Mischvorganges und dem Beginn der Prüfung ganz exakt eingehalten werden.

Für den eigentlichten Prüfvorgang kann zunächst die Nadel 12 genau auf die Oberfläche der im Prüfgefäß 8 befindlichen Probe eingestellt werden, wobei ein Höhenausgleich durch allfälliges Anheben des Fußteiles 2 oder Absenken des Auslegers 3, der dann am Gehäuse entsprechend zu führen wäre, vorgenommen werden kann. Nach einer bevorzugten Vorgangsweise wird die Nadel 12 um ein vorgegebenes Maß exzentrisch zur Achse des Tellers 7 eingestellt. Die Einstellung erfolgt durch den gesteuerten Kolbentrieb 5. Nun wird von der Steuereinrichtung 17 gesteuert der Auslöser 14 ausgeklinkt, so daß die Nadel das erste Mal im freien Fall von der Oberfläche her in die im Prüfgefäß 8 befindliche Probe eindringt. Die Eindringtiefe wird mittels der Meßeinrichtung 16 erfaßt, die entsprechenden Daten werden in der Auswerteeinheit 17 gespeichert. Nun steuert die Steuereinrichtung 17 das Anheben des Nadelhalters 10 über 13; sobald die Nadel die Probe verläßt, wird der Teller 7 mittels des Motors um einen vorgewählten Schritt - bei einer Nadeldicke von 1mm beispielsweise 3 mm - gedreht und im gewählten Zeittakt der Nadelhalter 10 wieder ausgelöst, so daß der nächste Einstich erfolgt. Die Daten werden wieder gespeichert. Sobald am ersten Kreis alle Einstiche durchgeführt sind, wird der Teller 7 mittels des Kolbentriebes 5 wieder verstellt, so daß die nächsten Einstiche auf einem konzentrischen äußeren Kreis erfolgen und so fort. Eine fertig geprüfte Probe 22 ist in Fig. 2 ersichtlich. Man sieht dort die auf konzentrischen Kreisen 23, 24, 25 liegenden Einstiche. Bei jedem Einstich wird die Eindringtiefe erfaßt und die entsprechenden Daten werden gespeichert. Die Zeitabstände zwischen aufeinanderfolgenden Einstichen können gleich groß oder nach einem vorgegebenen Programm auch variierbar sein. Man kann etwa für als besonders kritisch erkannte Erstarrungszeiten im Programm häufigere Einstiche als in den anderen Zeiten vorsehen. In der Praxis kann der Zeittakt von 3 Sekunden aufwärts variiert werden. Am Ende der Prüfung ist im Speicher des Computers 17 ein Datenprofil gespeichert, aus dem die Eindringtiefen in Zuordnung zur Zeit entommen werden können. Ein aus den Daten abgeleitetes Penetrations-Zeitdiagramm, das damit auch eindeutige Aussagen über die Erstarrungsgeschwindigkeit enthält, kann auf einem Bildschirm 26 der Steuer- und Auswerteeinheit 17 dargestellt oder auch mit Hilfe eines an diese Einheit angeschlossenen Druckers 27 ausgedruckt werden. Im Auswerteprogramm kann auch vorgesehen werden, daß Proben, die bestimmte grundsätzliche Bedingungen nicht erfüllen, bei denen also beispielsweise nach einer vorgewählten Zeitspanne ein vorgegebener Schwellwert der Eindringtiefe überschritten wird und damit die Erstarrung zu dieser Zeitspanne noch nicht den erwünschten Wert erreicht hat, durch zusätzliche Anzeigen in einer raschen Vorausbewertung als "schlecht" bewertet werden. Eine solche Schnellbewertung kann insbesondere bei der Überprüfung laufender Chargen wesentlich sein.

## Patentansprüche

1. Verfahren zum Prüfen des Erstarrungsverhaltens bzw. der Erstarrungsgeschwindigkeit von zementgebundenen Massen, insbesondere bei der Herstellung von Spritzbeton, bei dem die Eindringtiefe einer mit vorgewählter Kraft an eine aus der zu prüfenden Masse gebildeten Probe (22) angestellten Penetrationsnadel (12) als Maß für den momentanen Erstarrungszustand verwendet wird und unter vergleichbaren Bedingungen hergestellte bzw. gewonnene Proben (22) in becherartigen Prüfgefäßen (8) in den Verstellbereich der Nadel (12) verbracht, die von oben in das Prüfgefäß (8) tauchende Nadel (12) in festgelegten Zeitabständen in abstandsweise vorgesehene Einstichstellen eindringen gelassen und die der Eindringtiefe entsprechenden Daten erfaßt werden, gekennzeichnet durch die Verwendung einer Vorrichtung, die unterhalb einer Vertikalführung (9) für einen die Penetrationsnadel (12) haltenden, durch sein Eigengewicht und wahlweise anbringbare Zusatzgewichte (11) belasteten Nadelträger (10) eine z. B. tellerförmige Stütze (7) für je ein becherartiges Prüfgefäß (8) aufweist, welche Stütze (7) horizontal gegenüber dem Nadelträger (10) einstellbar und um ihre Achse verdrehbar ist, wobei für den Nadelträger (10) eine Hubeinrichtung (13) und eine Auslöseeinrichtung (14) zur Freigabe seiner freien Abwärtsbewegung sowie eine Wege-Meßeinrichtung (15, 16), für die Stütze (7) Antriebseinrichtungen (5, 6) zu ihrer Drehverstellung und Horizontaleinstellung vorgesehen sind und eine mit einem Rechner, insbesondere einem frei programmierbaren Personalcomputer ausgestattete Steuer- und Auswerteeinheit (17) die Hubeinrichtung (13), Auslöseeinrichtung (14) und Antriebseinrichtungen (5, 6) zyklisch steuert, so daß die Nadel (12) bei einem Zyklus im freien Fall in eine in dem auf der Stütze (7) befindlichen Prüfgefäß (8) enthaltene Probe (22) eindringt, die Eindringtiefe über die Wege-Meßeinrichtung (15, 16) von der Auswerteeinheit (17) erfaßt, die Nadel (12) angehoben, das Prüfgefäß (8) über die Antriebseinrichtungen (5, 6) verstellt und dann die Nadel (12) im Zeittakt neuerlich ausgelöst wird, so daß sie beim nächsten Zyklus in eine im Abstand von der letzten Einstichstelle befindliche Einstichstelle der Probe (22) eindringt, die Probe (22) nach Abschluß der Untersuchung in einem vorbestimmten Muster, z. B. mit gleichen Abständen auf konzentrischen Kreisen (23, 24, 25), Einstichstellen aufweist und in der Auswerteeinheit (17) ein auswertbares Datenprofil für die erfaßten Eindringtiefen gespeichert ist.

## Claims

1. A method of testing the setting behaviour or setting speed of cement-bonded materials, more particularly in the production of sprayed concrete, in which the depth of penetration of a penetration needle (12), set with a preselected force against a specimen (22) formed from the material under test, is used as an index of the instantaneous setting condition and specimens (22) obtained or made under comparable conditions are brought, in beaker-like test vessels (8), into the range of movement of the needle (12), the needle (12) entering the test vessel (8) from above is allowed to penetrate at fixed intervals of time into spaced penetration points, and the data corresponding to the penetration depth are determined, characterised by the use of a device which comprises, beneath a vertical guide (9) for a needle carrier (10) holding the penetration needle (12) and loaded by its own weight and optionally applied additional weights (11), a support (7), for example in the form of a plate, for each beaker-shaped test vessel (8), the support (7) being adjustable horizontally relatively to the needle carrier (10) end being rotatable about its axis, there being provided for the needle carrier (10) a lifting device (13) and a release device (14) to release its free downward movement and a travel measuring device (15, 16), drive means (5, 6) for the support (7) for rotation thereof and its horizontal adjustment, end a control and evaluation unit (17) equipped with a computer, more particularly a freely programmable personal computer, which cyclically controls the lifting device (13), release device (14) and drive means (5, 6) so that the needle (12) in one cycle in free fall penetrates into a specimen (22) contained in the test vessel (8) disposed on the support (7), the depth of penetration is detected by the evaluation unit (17) by way of the travel measuring device (15, 16), the needle (12) is lifted, the test vessel (8) is moved by way of the drive means (5, 6) and then the needle (12) is again released in the cyclic timing so that during the next cycle it penetrates into a penetration point of the specimen (22) spaced from the last penetration point, the specimen (22) after conclusion of the examination has penetration points in a predetermined pattern, e.g. at equal distances over concentric circles (23, 24, 25), and an evaluatable data profile for the penetration depths detected is stored in the evaluation unit (17).

## Revendications

1. Procédé pour tester le comportement ou la vitesse de solidification de masses liées par du ciment, en particulier lors de la fabrication de béton à projeter, pour lequel la profondeur de pénétration d'une aiguille (12) de pénétration, appliquée avec une force sélectionnée d'avance sur une éprouvette (22) formée à partir de la masse à tester, est utilisée comme mesure de l'état instantané de la solidification et des éprouvettes (22),confectionnées ou prélevées dans des conditions comparables, sont placées dans des vases d'essai (8) du genre godets dans le périmètre de déplacement de l'aiguille (12) pour laisser pénétrer par le haut, à des intervalles définis dans le temps, en des points de pénétration prévus pour les différents intervalles, l'aiguille (12) plongeant dans le vase d'essai (8) et saisir les données correspondant à la profondeur de pénétration,
caractérisé par l'utilisation d'un dispositif qui présente, en contre-bas d'un système (9) de guidage vertical destiné à un porte-aiguille (10) qui, maintenant l'aiguille (12) de pénétration, est placé sous la charge de son propre poids et de poids (11) d'appoint pouvant être ajoutés au choix, un support (7) par exemple en forme de plateau prévu pour un vase d'essai (8) en forme de godet à la fois, support (7) qui peut être repositionné dans le sens horizontal par rapport au porte-aiguille (10) et être tourné autour de son axe, tandis qu'il est prévu pour le porte-aiguille (10) un dispositif (13) de remontée et un dispositif (14) de libération autorisant son libre mouvement de descente, ainsi qu'un dispositif (15, 16) de mesure de déplacement pour le support (7) des dispositifs (5,6) d'entraînement pour le repositionnement dans le sens de sa rotation et dans le sens horizontal, et qu'une unité (17) d'exploitation équipée d'un ordinateur, en particulier d'un ordinateur individuel librement programmable, pilote cycliquement le dispositif (13) de remontée, le dispositif (14) de libération et les dispositifs (5, 6) d'entraînement, si bien que dans un premier cycle, l'aiguille (12) pénètre en chute libre dans une éprouvette (22) contenue dans le vase d'essai (8) se trouvant sur le support (7), la profondeur de pénétration est saisie par l'unité (17) d'exploitation par l'intermédiaire du dispositif (15, 16) de mesure de déplacement, l'aiguille (12) remontée, le vase d'essai (8) repositionné par l'intermédiaire des dispositifs (5, 6) d'entraînement, puis l'aiguille (12) à nouveau libérée à la cadence dans le temps si bien que dans le cycle suivant, elle pénètre en un point de pénétration se trouvant à distance du point de pénétration précédent sur l'éprouvette (22), que l'éprouvette (22) présente au terme de l'examen des points de pénétration répartis selon un dessin prédéterminé, par exemple à des écarts égaux sur des cercles (23, 24, 25) concentriques, et qu'un profil numérique de données exploitables des profondeurs de pénétration saisies est mémorisé dans l'unité (17) d'exploitation.
